# EUROPEAN PATENT APPLICATION

(11) **EP 2 213 265 A1**
(43) Date of publication of application: **04.08.2010**
(21) Application number: 08851084.7
(22) Date of filing: 18.11.2008
(51) Int. Cl.: A61F 5/56

(54) **AIRWAY-OPENING DEVICE**

(30) Priority: 22.11.2007 JP 2007303615; 12.09.2008 JP 2008235737
(71) Applicant: Nagasaki University, Nagasaki-Shi Nagasaki , 8528521 (JP)
(72) Inventor: AYUSE, Takao, Nagasaki-shi Nagasaki 852-8521 (JP); OI, Kumiko, Nagasaki-shi Nagasaki 852-8521 (JP); ISHIMATSU, Takakazu, Nagasaki-shi Nagasaki 852-8521 (JP); MOROMUGI, Shunji, Nagasaki-shi Nagasaki 852-8521 (JP)
(74) Representative: Müller - Hoffmann & Partner
(86) International application number: PCT/JP2008/070891
(87) International publication number: WO 2009/066645

(57) **Abstract**

The present invention provides an airway securing device which more sufficiently secures the airway to enable resolution of snoring, prevention and treatment of sleep apnea syndrome, and other airway securing. The airway securing device of the present invention is provided with a wearable body 3 integrally including a mechanism 5 to close the mouth and elevate the jaw tip as needed.

## Description

### Technical Field

The present invention relates to an airway securing device (airway-opening device) for treatment or symptom improvement of people suffering from snoring or sleep apnea syndrome, or for airway securing during a surgical operation.

### Background Art

Loudly snoring during sleep is not only annoyance to those around but also leads to one's bad sleep. It is reported that about 40% of adult male and about 15% of female adult are aware of snoring, and the way to resolve snoring is drawing a great attention, however, a decisive treatment has not been found yet.

The sleep apnea syndrome is a condition that the airway is closed and temporary apnea and/or hypopnea is repeated during sleep. It is said that the sleep apnea syndrome causes poor physical condition, daytime impairment of concentration, falling asleep at the wheel, etc., and further, it has been reported in a medical research as well that the level of oxygen in the body temporarily decreases due to the sleep apnea syndrome and that such decrease of the level of oxygen is a remote cause of high blood pressure and cardiac disease. Therefore, recently, attention is focused on the methods for preventing and treating the sleep apnea syndrome. Researches for treatment and symptom improvement of the sleep apnea syndrome are being carried out, such as diet treatment, physical condition maintenance, suitable shapes of pillows, etc., however, decisive treatment methods have not been found yet.

It is regarded as that one of the major causes of snoring and apnea and/or hypopnea is that in a condition of being on one's back, the muscles are relaxed, the mouth is half-opened, and the lower jaw drops backward, and thereby the tongue drops backward to close the airway. As a representative treatment method, a treatment method is known in which an oral brace called a "splint" is worn in the mouth during sleep to fix the tongue and the lower jaw frontward, and thereby the airway space located posterior to the tongue is expanded and closure of the airway is prevented (see Patent Document 1). Also, the CPAP treatment is available, in which air is forcibly sent into the airway through a single purpose nose mask to apply a positive pressure (see Patent Document 2). Further, a pillow which forces lying on one's side during sleep (see Patent Documents 2, 3), a shoulder pillow which tilts the head backward (see Patent Document 4), etc. have been known.

Patent Document 1: Japanese Unexamined Patent Application Publication No. 2005-95218
Patent Document 2: Japanese Unexamined Patent Application Publication No. 2006-231008
Patent Document 3: Japanese Unexamined Patent Application Publication No. 2005-118241
Patent Document 4: Japanese Unexamined Patent Application Publication No. 2006-101934

### Disclosure of the Invention

### Problems to be Solved by the Invention

Meanwhile, problems remain that the above-described splint cannot be worn when the teeth are defective, such as when the teeth have cavities or pyorrhea, and that although the CPAP treatment is effective for medium to serious cases, it is unsuitable for relatively light cases. The instrument for preventing snoring using a pillow which forces lying on one's side has an effect on alleviating closure of the airway, which, however, is not sufficient, and moreover, because the patient lies on one's side during sleep, an extreme body posture is forced, and there is a possibility that numbness of arms, etc. are induced. The instrument for preventing snoring using a shoulder pillow which tilts the head backward also has an effect on alleviating closure of the airway, however, the mouth tends to be in an open state and the airway cannot be sufficiently secured, and moreover, an uncomfortable feeling is given to the patient, for example, the mouth is dried.

Further, for example, also when airway securing is necessary during sleep due to anesthesia during an operation, it is desired that the airway can be secured by a simple device.

The present invention provides, in view of the above-described respects, an airway securing device that secures the airway more sufficiently, without forcing a sleeping body posture or giving an uncomfortable feeling, and enables treatment and symptom improvement of snoring and sleep apnea syndrome, other airway securing, etc.

### Means of Solving the Problems

An airway securing device according to the present invention is provided with a wearable body having a mechanism to close the mouth and elevate the jaw tip.
A preferable embodiment of the airway securing device of the present invention includes in the wearable body a mechanism to tilt the head backward.
A more preferable embodiment of the airway securing device of the present invention includes in the wearable body a mechanism to detect an abnormality in the patient during sleep.
A more preferable embodiment of the airway securing device of the present invention includes, as the mechanism to detect an abnormality in the patient during sleep, either or both of a device to detect apnea and/or hypopnea and a device to detect occurrence of snoring.

In the airway securing device of the present invention, during sleep or in falling asleep, a mechanism to close the mouth and elevate the jaw tip is driven as needed, and thereby the mouth of the patient in sleep is closed and the jaw tip is elevated. Further, when a mechanism to tilt the head backward is included, backward tilting of the head is carried out by driving of this mechanism, so that the airway space is further expanded, and airway securing is more reliably carried out.
For example, when snoring or apnea and/or hypopnea has been detected by the mechanism to detect an abnormality, the mechanism to close the mouth and elevate the jaw tip is driven based on detect signals thereof, and the mouth of the patient in sleep is closed and the jaw tip is elevated. Further, as needed, the mechanism to tilt the head backward is driven, and backward tilting of the head is carried out. Thereby, the airway space is expanded, and the airway can be secured.

Also, an airway securing device of the present invention is provided with a wearable body having a pouch-like body on a front upper surface of a collar-like body thereof, the pouch-like body being inflated and deflated by air and serving as a mechanism to elevate the jaw tip.
A preferable embodiment of the airway securing device of the present invention includes a pouch-like body, which is inflated and deflated by air and which serves as a mechanism to tilt the head backward, at a rear back surface of the collar-like body.
A more preferable embodiment of the present invention includes in the collar-like body a mechanism to detect an abnormality in the patient during sleep.
For example, when snoring or apnea and/or hypopnea has been detected by the mechanism to detect an abnormality, based on detect signals thereof, the pouch-like body as the mechanism to close the mouth and elevate the jaw tip is driven, and the mouth of the patient in sleep is closed and the jaw tip of the patient is elevated. Further, as needed, the pouch-like body as the mechanism to tilt the head backward is driven, and thereby backward tilting of the head is carried out. Thereby, the airway space is expanded, and the airway can be secured.

In the airway securing device according to the present invention, during sleep or when falling asleep, the pouch-like body as the mechanism to close the mouth and elevate the jaw tip is driven as needed, and the mouth of the patient in sleep is closed and the jaw tip of the patient is elevated. Further, when the pouch-like body as the mechanism to tilt the head backward is included, backward tilting of the head is carried out by driving of the pouch-like body, and the airway is more reliably secured by further expansion of the airway space.

Also, an airway securing device according to the present invention includes a lower frame and an upper frame axially supported at respective both ends so as to open and close, the upper frame touching the lower jaw when the device is worn on the patient, a first pulley and a second pulley fixed at both ends of the upper frame, and a drive transmitting body which is fixed at one end thereof to the first pulley and wound around the first pulley and the second pulley, and which opens and closes the upper frame relative to the lower frame by a pushing and pulling operation thereof.

The airway securing device of the present invention is worn on the front collar portion of the patient in sleep in a state that the upper frame is closed relative to the lower frame. And, by driving the drive transmitting body to be pulled from the outside of the frames, the second pulley and the first pulley are rotated at the same time, so that the upper frame which is integral with the first pulley and the second pulley are rotated to open relative to the lower frame. Because the upper frame opens while being in contact with the lower jaw, the jaw tip is elevated, and the airway space is expanded, so that the airway can be secured.

According to the airway securing device according to the present invention, the mouth is closed and the jaw tip is elevated by driving of the mechanism to close the mouth and elevate the jaw tip, and thereby the airway is secured, so that treatment and symptom improvement of snoring and sleep apnea syndrome, and other airway securing can be carried out. Because the present airway securing device is worn on the body, the sleeping body posture will be never forced (fixed), and an uncomfortable feeling will be never given to the patient.

Further, according to the airway securing device according to the present invention, the upper frame is opened while being in contact with the lower jaw, and the jaw tip is elevated, and thereby the airway is secured, so that treatment and symptom improvement of snoring and sleep apnea syndrome, and other airway securing can be carried out. Further, the body posture during sleep will be never forced, and an uncomfortable feeling will be never given to the patient.

### Brief Description of Drawings

FIG. 1 is a schematic configuration diagram illustrating the first embodiment of an airway securing device according to the present invention.
FIG. 2A, FIG. 2B, and FIG. 2C are diagrams illustrating the first operation, the second operation, and the third operation of the airway securing device of the first embodiment.
FIG. 3 is a schematic configuration diagram illustrating the second embodiment of the airway securing device according to the present invention.
FIG. 4A and FIG. 4B are diagrams illustrating the first operation and the second operation of the airway securing device of the second embodiment.
FIG. 5A is a perspective view illustrating another example of a pouch-like body which elevates the jaw tip to have the mouth closed according to the present invention, and FIG. 5B is a partially cross-sectional state diagram illustrating a state of elevating the jaw tip.
FIG. 6 is a schematic configuration diagram of the airway securing device according to the present invention when a belt-type sensor and a microphone have been concurrently used for the sensor.
FIG. 7 is a schematic configuration diagram of the airway securing device according to the present invention when only a microphone has been used for the sensor.
FIG. 8A and FIG. 8B are schematic configuration diagrams illustrating the third embodiment of the airway securing device of the present invention when viewed at a normal time from the top and from a side.
FIG. 9A and FIG. 9B are schematic configuration diagrams illustrating the third embodiment of the airway securing device of the present invention when viewed at an abnormal time from the top and from a side.
FIG. 10 is a schematic configuration diagram illustrating the fourth embodiment of the airway securing device according to the present invention.
FIG. 11 is a partially cross-sectional side view of the major part of the airway securing device of the fourth embodiment.
FIG. 12 is a configuration diagram illustrating a support frame portion of the airway securing device of the fourth embodiment.
FIG. 13 is an explanatory diagram illustrating a worn state at a normal time of the airway securing device of the fourth embodiment.
FIG. 14 is an explanatory diagram illustrating a state of the airway securing device of the fourth embodiment that the lower jaw elevating operation has been carried out.
FIG. 15 is a schematic diagram illustrating a state of the airway securing device of the fourth embodiment, in which the frame has been closed.
FIG. 16A and FIG. 16B are explanatory diagrams for explaining the operations of a pulley on the side of the arm portion 33b on one side and a pulley on the side of the arm 33a on the other side of the airway securing device of the fourth embodiment when the upper frame is closed.
FIG. 17 is a schematic diagram illustrating a state of the airway securing device of the fourth embodiment, in which the frame has been opened.
FIG. 18A and FIG. 18B are explanatory diagrams for explaining the operations of the pulley on the side of the arm portion 33b on one side and the pulley on the side of the arm portion 33b on the other side of the airway securing device of the fourth embodiment when the upper frame is opened.

### Best Mode for Carrying Out the Invention

Below, embodiments of the present invention will be described referring to drawings.

### [First Embodiment]

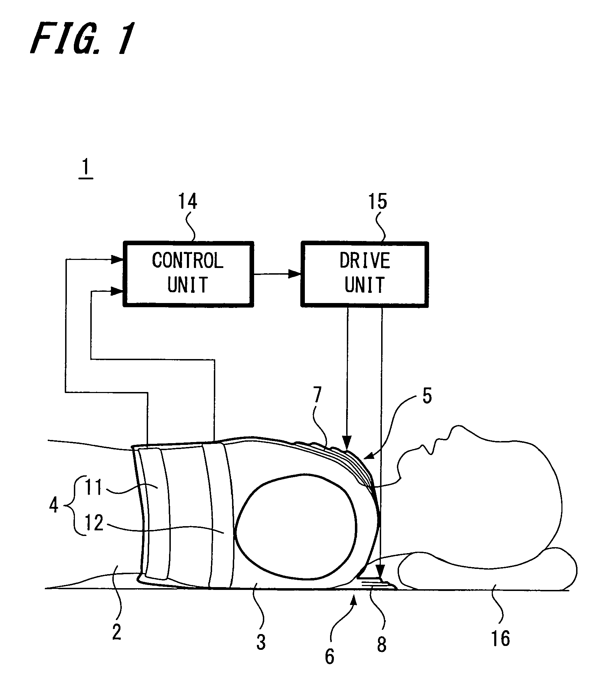

FIG. 1 illustrates the first embodiment of an airway securing device according to the present invention. In the present embodiment, an example is illustrated in which the present airway securing device has been applied to a treatment apparatus for snoring and sleep apnea syndrome. An airway securing device 1 according to the present embodiment includes a wearable body 3 having a mechanism 5 which closes the mouth and elevates the jaw tip and preferably a mechanism 6 which tilts the head backward. The mechanism 5 closing the mouth and elevating the jaw tip and the mechanism 6 tilting the head backward are preferably configured to be integrally attached to the wearable body 3, however, may be configured separate from the wearable body 3 or attachable to and detachable from the wearable body 3, and arranged in or attached to the wearable body 3 at the time of use. Further, preferably, the wearable body 3 includes a mechanism which detects an abnormality in the patient during sleep. It is preferable that the mechanism which detects an abnormality is also configured to be integrally attached to the wearable body 3, however, may be configured separate from the wearable body 3 or attachable to and detachable from the wearable body 3, and arranged in or attached to the wearable body 3 at the time of use. The mechanism which detects an abnormality in the patient during sleep integrally includes either or both of a device to detect apnea and/or hypopnea and a device to detect occurrence of snoring. In the present example, for a detection device to detect snoring or apnea and/or hypopnea, a sensor 4 is used. The wearable body 3 is worn on a body (i.e., patient) 2. Further, the present airway securing device 1 includes a control unit 14 and a drive unit 15, which are described later. The drive unit 15 drives the mechanism 5 and the mechanism 6, and the control unit 14 determines whether snoring or apnea and/or hypopnea is present based on signals obtained at the sensor 4, and sends instructions to the drive unit 15.

For the wearable body 3, in the present example, clothing, in particular, a vest, is used. At a position of the vest below the chin, namely, at a front collar portion of the vest, an air chamber which is inflated and deflated by air, a so-called pouch-like body 7, is provided to constitute the mechanism 5 which closes the mouth and elevates the jaw tip. The pouch-like body 7 may be formed by a light and flexible material, for example by a vinyl material. The mechanism 5 which closes the mouth and elevates the jaw tip is formed by four bags communicating and coupled with one another, and is in a deflated condition at a normal time at the front collar portion of the vest as the wearable body 3. Once snoring or apnea and/or hypopnea is confirmed, the pouch-like body at the front collar of the vest is filled by air to be inflated, and bulges toward the jaw, thereby pushing the chin, and as a result, the mouth of the user is closed and the jaw tip is elevated. In the illustrated example, the mechanism 5 to close the mouth and elevate the jaw tip is configured by four pouch-like bodies coupled with one another, however, may be configured, for example, by a single pouch-like body, and thus the mechanism 5 to close the mouth and elevate the jaw tip may be configured by various numbers and arrangements of pouch-like bodies.

The mechanism 6 which tilts the head backward is formed, in the present example, by an air chamber which is inflated and deflated by air, a so-called pouch-like body 8. The pouch-like body 8 may be formed by a light and flexible material, for example, by a vinyl material. The pouch-like body 8 is formed like a pillow, and is attached to the position in the back of the vest corresponding to the shoulder, that is, to the rear collar portion of the vest. The pouch-like body 8 is configured to be inflated by air to elevate the shoulder or chest and tilt the head backward.

For the sensor 4, an existing sensor may be used, such as a sensor which detects inspiration and/or expiration and apnea based on swelling patterns of the abdomen and the chest, a microphone which detects a snoring sound and a respiratory sound, a sheet-like pressure distribution profiler which detects a breathing-related change in the pressure distribution between the body trunk and the bed, or a thermistor which is attached to the nose (to be exact, on the lip roughly below the nostrils) and detects inspiration and/or expiration and apnea. The thermistor detects whether expiration is present by converting the temperature change to a breathing pattern. Of course, it is possible to use a sensor which can detect both whether expiration is present and whether snoring is present.

In the present embodiment, among those sensors, a sensor which detects whether expiration is present by detecting swelling patterns in the abdomen and the chest is integrally incorporated in the vest as the wearable body 3. The sensor 4 of the present example includes a belt-type sensor unit 11 and a similar sensor unit 12, both including a resistance wire or conductive rubber and capable of detecting expansion and contraction amounts using the resistance wire or conductive rubber.

It is determined whether apnea and/or hypopnea is present based on swelling patterns of the abdomen and the chest obtained through the sensor 4. When breathing is normal, the swelling patterns of the abdomen and the chest are in the same phase. However, in a state that the airway is closed and breathing has stopped, the swelling patterns of the abdomen and the chest are in opposite phases. Accordingly, by detecting such opposite patterns, the apnea and/or hypopnea condition can be detected. Note that in FIG. 1, numeral 16 denotes a normal pillow.

Further, the airway securing device 1 of the present embodiment includes the control unit 14 determining whether apnea and/or hypopnea is present based on signals detected by the sensor 4 and based on determined results and drive controlling the mechanism 5 to close the mouth and elevate the jaw tip and the mechanism 6 to tilt the head backward, and the drive unit 15 driving the mechanism 5 to close the mouth and elevate the jaw tip and the mechanism 6 to tilt the head backward based on instructions from the control unit 14.

When the mechanism 5 and the mechanism 6 are configured by the above-described pouch-like bodies 7 and 8, the drive unit 15 includes a mechanism to inflate and deflate the pouch-like bodies 7 and 8 by air. That is, the drive unit 15 is provided with a function to supply air to the mechanism 5 and the mechanism 6 when a symptom appears, and a function to deflate the mechanism 5 and the mechanism 6 when the condition has returned normal. As the deflating mechanism, for example, a mechanism to release a valve to discharge air, a mechanism to suck in air by applying negative pressure, and other mechanisms are possible. The pouch-like bodies 7 and 8 may be configured to be mechanically contracted. For the drive unit 15, preferably, a small electric air pump may be used for sucking air, and a small electromagnetic valve may be used for discharging air. It is believed that the electric air pump will sufficiently function even though it is the one for low pressure which operates by a battery.

Further, as the mechanism to inflate and deflate the pouch-like bodies 7 and 8 by air, for example, it may be configured such that a valve (for example, a valve for switching air supplying and exhausting, a special air supplying valve and a special air exhausting valve, or a valve exclusively for air exhausting, etc.) is arranged, and an elastic member, which always elastically biases the pouch-like bodies 7 and 8 in the contracting direction, for example, an elastic rubber belt, is attached to the wearable body 3. When inflating the pouch-like bodies 7 and 8, air is injected against the elastic and biasing force of the elastic member to inflate the pouch-like bodies 7 and 8. When contracting the pouch-like bodies 7 and 8, the valve is released, and the pouch-like bodies 7 and 8 are contracted by the elastic and biasing force of the elastic member.

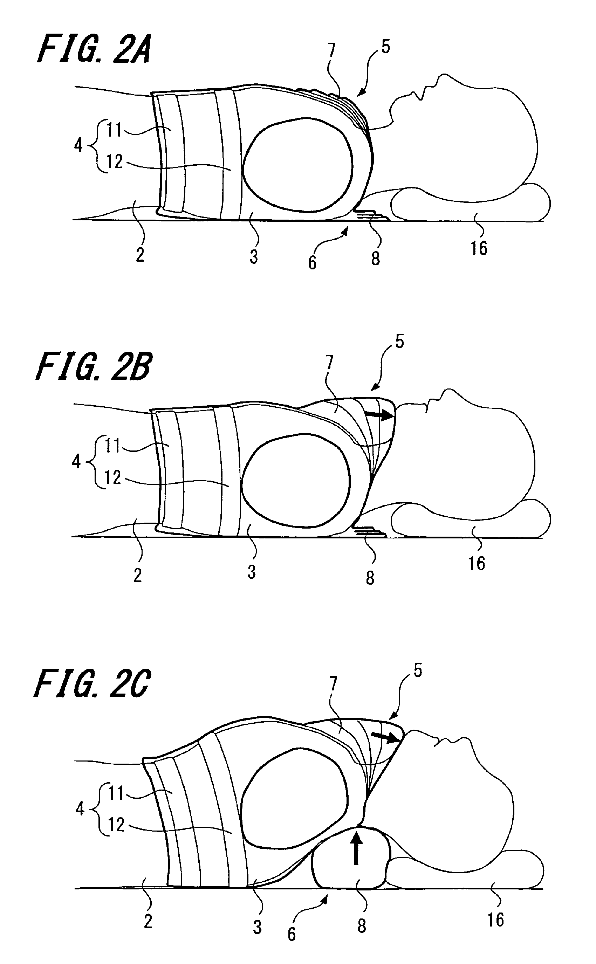

Next, the operation of the airway securing device 1 according to the first embodiment is described using FIG. 2A, FIG. 2B, and FIG. 2C. The patient falls asleep wearing the wearable body 3, i.e., the vest of the airway securing device 1. FIG. 2A, FIG. 2B, and FIG. 2C illustrate a state that the patient lies on one's back with the head on a pillow 16.

FIG. 2A illustrates a state at a normal time. At this time, the pouch-like body 7 at the front collar portion, which is the mechanism 5 to close the mouth and elevate the jaw tip, and the pouch-like body 8 at the rear collar portion, which is the mechanism 6 to tilt the head backward, are in contracted conditions, respectively.

Next, in a case that the airway securing device 1 is used by a patient suffering from sleep apnea syndrome, if the patient turns into a state of stoppage of breathing, the breathing pattern from the belt-type abdomen sensor unit 11 and the breathing pattern from the belt-type chest sensor unit 12 are in opposite phases, so that based on the phase relationship between these breathing patterns, whether the patient is breathing is determined at the control unit 14. When it is determined as that the breathing is stopped, based on the determination result, according to an instruction from the control unit 14, the drive unit 15 starts driving, and first, air is sent from the drive unit 15 to the pouch-like body 7 at the front collar portion.

Thereby, as illustrated in FIG. 2B, the pouch-like body 7 is inflated, the mouth of the patient is closed and the jaw tip of the patient is elevated. Because the mouth is closed and the jaw tip is elevated, the airway space of the patient is expanded. In this state, the airway is secured depending on the symptom of the patient, and breathing returns normal.

Further, as illustrated in FIG. 2C, after the pouch-like body 7 at the front collar portion has been inflated, air is sent from the drive unit 15 to the pouch-like body 8 at the rear collar portion. Then, the pouch-like body 8 is inflated, and thereby the neck or a part of the chest of the patient is elevated, so that the head tilts backward. Due to backward tilting of the head, the airway space of the patient is further expanded, and thereby the airway is reliably secured, so that breathing returns normal.

If the sensor 4 detects that breathing has returned normal, it is determined at the control unit 15 that breathing is normal. Based on the determination result, according to an instruction from the control unit 14, exhausting driving is carried out at the drive unit 15. Thereby, the pouch-like bodies 7 and 8 are deflated, and contracted to return normal, and as a result, the patient returns to the state of FIG. 2A.

As the operational control to the pouch-like bodies 7 and 8 after stoppage of breathing has been detected, as described above, upon detecting stoppage of breathing, the pouch-like bodies 7 and 8 are inflated by sending air thereto, and upon detecting start of breathing, the pouch-like bodies 7 and 8 are deflated to be contracted. In addition, it is also possible to send air to the pouch-like bodies 7 and 8 upon detecting stoppage of breathing, maintain the shapes of the inflated pouch-like bodies 7 and 8 for a certain period of time, and anticipating that the airway is secured during that period of time, deflate and contract the pouch-like bodies 7 and 8 after the certain period of time. Further, depending on the symptom of the patient, it is possible to operate only the pouch-like body 7 or both of the pouch-like bodies 7 and 8. Thus, the manner of drive controlling the pouch-like bodies 7 and 8 can be variously selected to suit symptoms of the patient. Based on each manner of driving control, control systems of the control unit 14 and the drive unit 15 are set.

According to the airway securing device 1 according to the above-described first embodiment, by wearing the wearable body 3 in which the pouch-like body 7 is attached to the front collar portion and the pouch-like body 8 is attached to the rear collar portion, during sleep, when apnea and/or hypopnea has been detected by the sensor 4, the pouch-like body 7 or both of the pouch-like bodies 7 and 8 are inflated, the mouth of the patient is closed and the jaw tip is elevated, and further, the head is tilted backward, and thereby the airway can be secured, and breathing can be restored.

The airway securing device 1 of the first embodiment will never enforce a body posture during sleep. That is, the patient can freely change the body posture, and the body posture will be never required to be fixed, so that burdens on the patient can be reduced. For example, in the case of an existing treatment instrument which requires the patient to lie on one' s side, the patient is fixed lying on one' side, so that it gives an uncomfortable feeling to the patient, for example, rolling over in bed is not possible, the arm on one side becomes numb, and so on, and in the case of a treatment instrument which carries out only tilting the head backward, because the mouth continues to be opened, the mouth dries, thus giving an uncomfortable feeling. However, the airway securing device 1 is improved in these respects as well.

The vest as the wearable body 3 and the pouch-like bodies 7 and 8 are all light and soft, so that an unnecessary feeling of pressure will not be given to the patient, and even such a patient who tosses around in bed can reliably operate the airway securing device 1. Also, the airway securing device 1 of the present embodiment can be carried, so that it can be used in the travel destination, etc. The structure of the pillow 16 is not regulated, so that a pillow of one' s choice can be used.

Note that in the above-described airway securing device 1, it is possible to drive only the pouch-like body 8 which tilts the head backward, however, in this case, there is a fear that an uncomfortable feeling is given to the patient, for example, the mouth is opened and dries, so that it is preferable to use the pouch-like body 8 concurrently with the pouch-like body 7 which elevates the jaw tip to have the mouth closed.
The above-described airway securing device 1 may be configured to integrally include only the pouch-like body 7 which elevates the jaw tip to have the mouth closed, or to integrally include only the pouch-like body 8 which tilts the head backward, or to integrally include both of the pouch-like bodies 7 and 8.

In the above-described airway securing device 1, the drive unit 15 has a function to limit the air pressure of the pouch-like bodies 7 and 8 not to rise any more, once necessary air has been supplied to the pouch-like bodies 7 and 8. When optimum values of the air pressure of the pouch-like body 7 at the front collar portion and the air pressure of the pouch-like body 8 at the rear collar portion are different, two drive units 15 can be provided.

In FIG. 2, it has been configured such that the pouch-like body 7 is driven first, and then the pouch-like body 8 is driven, however, it is also possible to drive the pouch-like bodies 7 and 8 at the same time, or to drive the pouch-like body 8 first and then drive the pouch-like body 7. Further, it is possible to drive only the pouch-like body 8. That is, the manner of drive control of the pouch-like bodies 7 and 8 can be selected to suit the symptom of the patient, including the above-described manner of drive control, and according to the selected manner of drive control, the control systems of the control unit 14 and the drive unit 15 are set.

### [Second Embodiment]

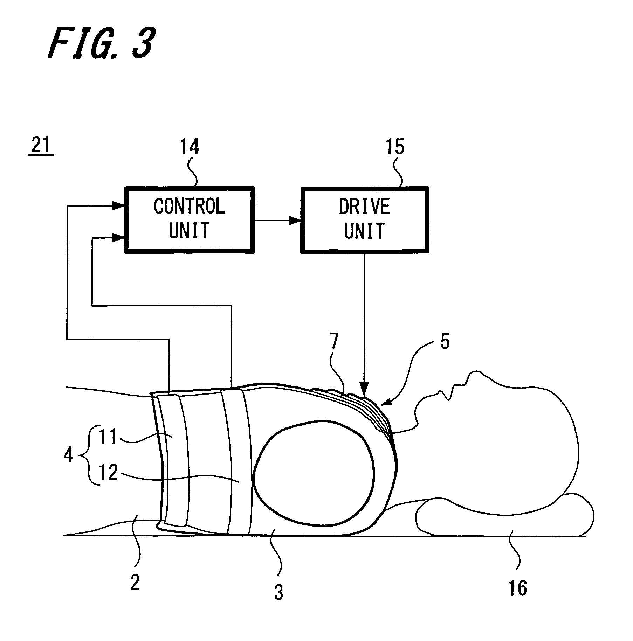

FIG. 3 illustrates the second embodiment of the airway securing device according to the present invention. The present embodiment is an example in which the airway securing device has been applied to a treatment apparatus for snoring and sleep apnea syndrome. An airway securing device 21 according to the present embodiment is configured by omitting the mechanism 6 which tilts the head backward in the airway securing device 1 of FIG. 1. That is, the airway securing device 21 according to the present embodiment includes a wearable body 3 integrally having a detect device detecting snoring or apnea and/or hypopnea, i.e., a sensor 4, and a mechanism 5 to close the mouth and elevate the jaw tip. Further, the airway securing device 21 includes a control unit 14 and a drive unit 15. The control unit 14 determines whether apnea and/or hypopnea or snoring is present based on signals detected by the sensor 4, sends the determination result to the drive unit 15, and drive controls the drive unit 15. The drive control unit 15 drives the mechanism 5 to close the mouth and elevate the jaw tip, based on instructions from the control unit 14.

In the present embodiment, similarly as explained referring to FIG. 1, clothing, especially, a vest is used for the wearable body 3. Also, the mechanism 5 to close the mouth and elevate the jaw tip is formed by a pouch-like body 7 which is inflated and deflated by air. The sensor 4 is constituted by sensor units 11 and 12 which detect breathing patterns of the abdomen and the chest. Further, the drive unit 15 has a function to supply air to the mechanism 5 when a symptom appears and a function to deflate the mechanism 5 when the condition has returned normal. The configuration of other parts is substantially the same as explained referring to FIG. 1, so that the overlapped explanation is omitted.

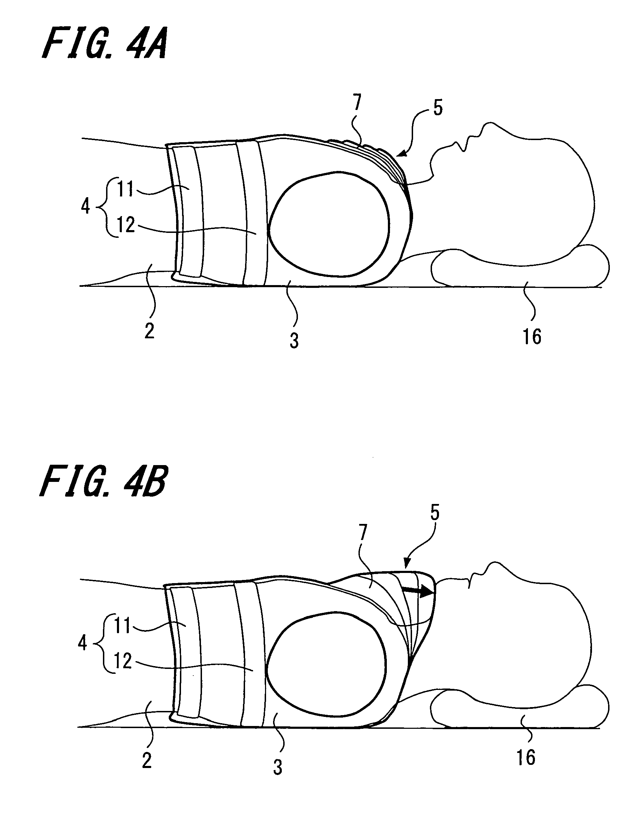

Next, the operation of the airway securing device 21 according to the second embodiment is described using FIG. 4A and FIG. 4B. The patient falls asleep wearing the wearable body 3 of the airway securing device 21. FIG. 4A illustrates a state at a normal time. At this time, the pouch-like body 7 at the front collar portion, serving as the mechanism 5 to close the mouth and elevate the jaw tip, is in a deflated condition.

Next, in a case that the airway securing device 21 is used by a patient suffering from sleep apnea syndrome, if the patient turns into a state of stoppage of breathing, the breathing pattern from the belt-type abdomen sensor unit 11 and the breathing pattern from the belt-type chest sensor unit 12 are in opposite phases, so that based on the phase relationship between these breathing patterns, whether breathing has stopped is determined at the control unit 14. And, based on the determination result, according to an instruction from the control unit 14, the drive unit 15 starts driving, and air is sent from the drive unit 15 to the pouch-like body 7 at the front collar portion.

Thereby, as illustrated in FIG. 4B, the pouch-like body 7 is inflated, the mouth of the patient is closed and the jaw tip is elevated. Because the mouth is closed and the jaw tip is elevated, the airway space of the patient is expanded. In this state, the airway is secured, and breathing returns normal.

According to the airway securing device 21 according to the second embodiment, similarly to the above-described example, by falling asleep while wearing the vest which is the wearable body 3 in which the pouch-like body 7 is attached to the front collar portion, during sleep, when the patient suffering from sleep apnea syndrome has turned into apnea and/or hypopnea, the pouch-like body 7 is inflated, and thereby the mouth is closed and the jaw tip is elevated, so that the airway can be secured, and breathing can be restored. In addition, effects similar to those explained with reference to the airway securing device 1 of the first embodiment can be obtained, such as that the body posture during sleep is not fixed, so that an uncomfortable feeling will not be given to the patient, and that carrying the device is possible, etc.

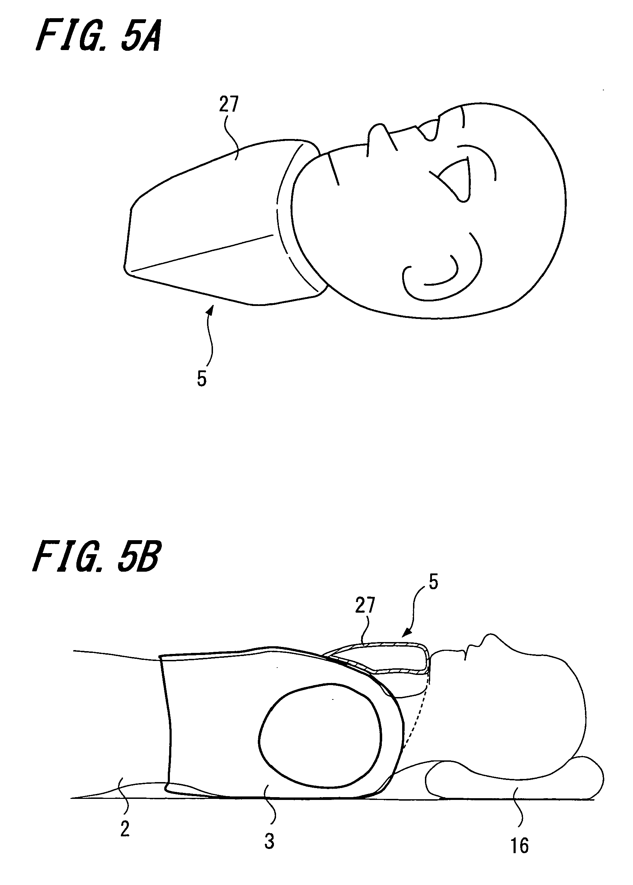

FIG. 5A and FIG. 5B illustrate another example of the pouch-like body at the front collar portion which is used for the mechanism 5 to close the mouth and elevate the jaw tip. A pouch-like body 27 is constituted by a bag formed of three parts adapted to the jaw tip and both sides of the jaw. The pouch-like body 27 is inflated and deflated in a vertical direction.

As the mechanism 5 to close the mouth and elevate the jaw tip, mechanisms other than the above-describe pouch-like body which is inflated and deflated by air may be possible. For example, the mechanism 5 may be configured such that one end of a frame is fixed to the front collar portion of the wearable body so as to stand. In this case, based on an on or off signal from the drive unit 14, the frame is caused to stand or lie, and when the frame stands, the frame pushes the jaw tip, and thereby the mouth is closed and the jaw tip is elevated.

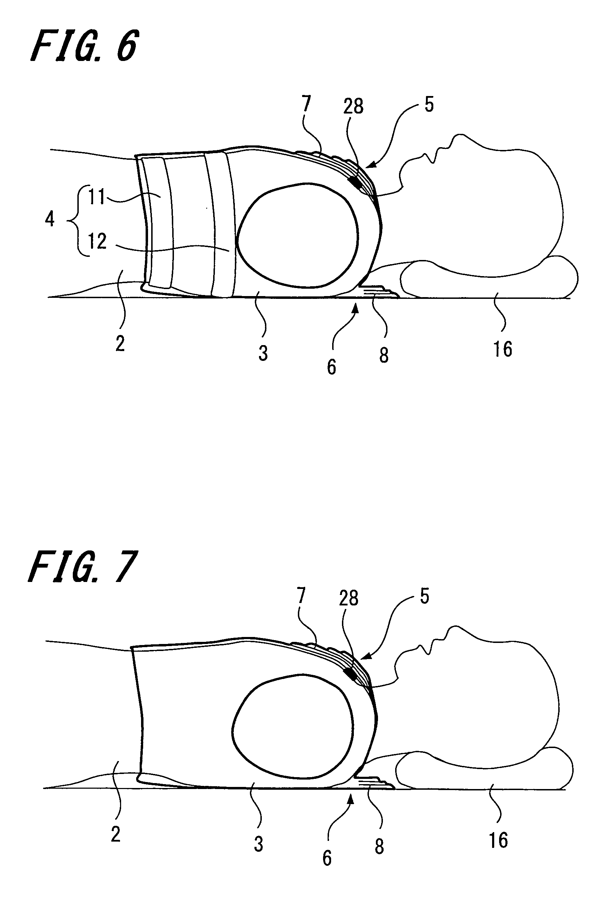

In the above-described example, the sensor 4 constituted by the belt-type sensor units 11 and 12 has been used, however, as illustrated in FIG. 6, detection of both of apnea and/or hypopnea and snoring can be enabled by attaching the belt-type sensor units 11 and 12 and a microphone 28 to the vest. Alternatively, as illustrated in FIG. 7, it is possible to detect both of apnea and/or hypopnea and snoring using only the microphone 28 for the sensor. In either case, the control unit 14 can be configured to deal with detecting both of snoring and apnea and/or hypopnea or detecting only snoring or apnea and/or hypopnea. In a case that treatment of sleep apnea syndrome is aimed, concurrently using a sensor for detecting snoring, such as a microphone etc. , in addition to using a sensor for detecting apnea and/or hypopnea, initiates the airway securing action based on snoring which appears as a preliminary step to apnea and/or hypopnea, so that it becomes possible to realize the operation of the device at an earlier stage.

In the above-described example, a vest is used for the wearable body 3, however, other waistcoats or sash-like body-wearable ones can be used.

### [Third Embodiment]

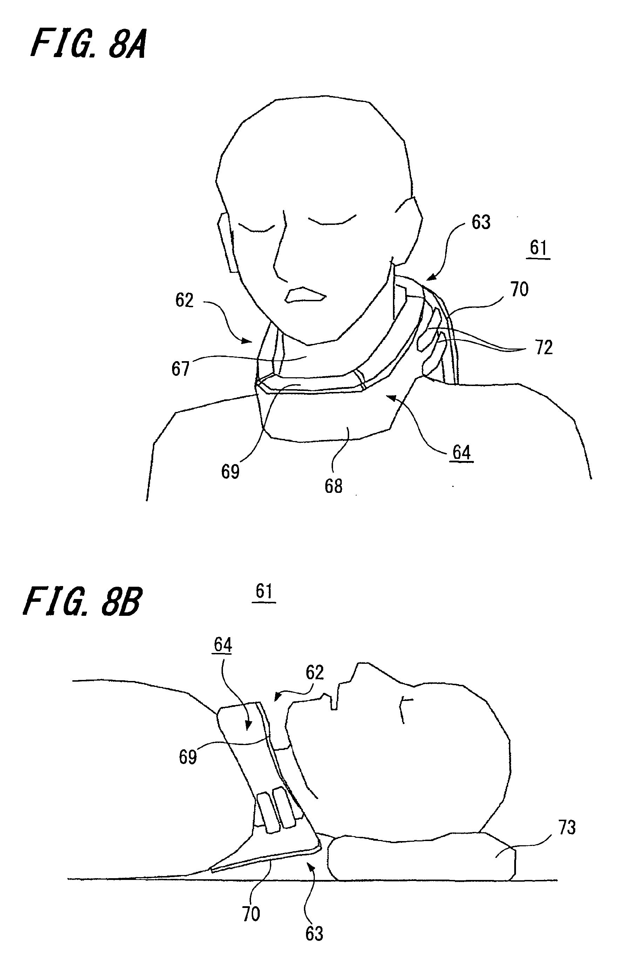

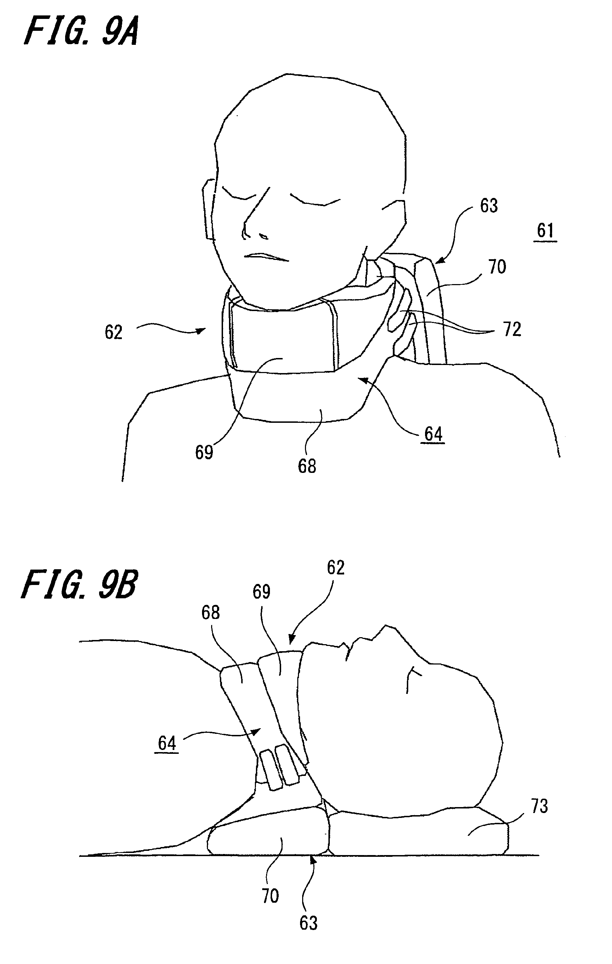

FIG. 8A, FIG. 8B, FIG.9A, and FIG. 9B illustrate the third embodiment of the airway securing device according to the present invention. An airway securing device 61 according to the present embodiment includes a wearable body 64 having a mechanism 62 which close the mouth and elevates the jaw tip, and preferably a mechanism 63 which tilts the head backward. The wearable body 64 includes a collar-like body 68 which can be detachably attached to a neck 67 of the patient with room, and an airbag 69 for elevating the lower jaw, which constitutes the mechanism 62, and an airbag 70 for tilting the head backward, which constitutes the mechanism 63, are arranged, respectively, at front and rear portions of the collar-like body 68 sandwiching the neck 67. It is preferable that the airbags 69 and 70 are integrally attached to the collar-like body 68, however, can be configured separate from the collar-like body 68 or attachable to and detachable from the collar-like body 68, and arranged in or attached to the collar-like body 68 at the time of use.

The airbags 69 and 70 can be each constituted by a pouch-like body which is inflated and deflated by air. The airbags 69 and 70 can be driven, for example, based on detection of an abnormality in the patient, such as snoring or apnea and/or hypopnea.

The collar-like body 68 is formed, for example, by a light foamable resin material, and is formed so as to open and close around an integrally molded joining portion. At opening and closing ends of the collar-like body 68, for example, two pieces of Velcro Tape (registered trademark) 72 are attached, and the collar-like body 68 is fixed in a closed state by the adhesive force of the Velcro Tape (registered trademark) 72.

The airbag 69 is integrally attached to a front upper surface of the collar-like body 68, which opposes the lower jaw, so as to be located at the front side of the neck 67, and the airbag 70 is integrally attached to a rear back surface of the collar-like body 68 so as to be located at the rear side of the neck 67. The airbags 69 and 70 are each formed by a pouch-like body which is inflated by being filled with air and contracted by being deflated, similarly as described earlier.

The wearable body 64 is used in combination with a mechanism to detect an abnormality in the patient during sleep. The mechanism to detect an abnormality integrally includes either or both of a detection device detecting apnea and/or hypopnea and a detection device detecting occurrence of snoring. For example, for the mechanism detecting an abnormality, a detect device detecting snoring and apnea and/or hypopnea can be used, such as a microphone, a sensor, and so forth. When using a microphone, the microphone may be integrally mounted on the collar-like body 68. The above-described sensor, etc. are to be arranged separately from the wearable body 64.

Further, the airway securing device 61 of the present embodiment includes, though not illustrated, a control device for drive controlling the airbag 69 as the mechanism 62 to close the mouth and elevate the jaw tip and the airbag 70 as the mechanism 63 to tilt the head backward, and a drive device for driving the airbags 69 and 70 under instructions of the control unit, which are similar to those described with reference to the first embodiment.

Next, the operation of the airway securing device 61 of the third embodiment is described. The airway securing device 61 is arranged, as illustrated in FIG. 8A, FIG. 8B, FIG. 9A, and FIG. 9B, such that the collar-like body 68 of the wearable body 64 is worn around the neck 67 with room in a state that the patient lies.

FIG. 8A and FIG. 8B illustrate a condition at a normal time. The patient lies on one's back with the head on a pillow 73. At this time, the airbags 69 and 70 are both in deflated conditions. Further, a space is formed between the airbag 70 at the rear of the neck 67 and the bed, corresponding to the height of the pillow 73. The space facilitates inflation of the airbag 70.

Next, if the patient goes into a state of apnea and/or hypopnea or snoring, based on detection of an abnormality from a detection device, an instruction is transmitted from the control unit to the drive unit, the drive unit starts driving, and first, air is sent from the drive device to the airbag 69. Thereby, as illustrated in FIG. 9A and FIG. 9B, the airbag 69 opposing the lower jaw is inflated, the mouth of the patient is closed and the jaw tip is elevated. Because the mouth is closed and the jaw tip is elevated, the airway space of the patient is expanded, the airway is secured, and breathing returns normal.

Further, after the airbag 69 has been inflated, air is sent from the drive unit to the airbag 70 at the rear of the neck, and the airbag 70 is inflated. Due to inflation of the airbag 70, the head is tilted backward. Because of backward tilting of the head, the airway space of the patient is further expanded, the airway is reliably secured, and normal breathing is restored.

According to the airway securing device 61 according to the third embodiment, based on detection of apnea and/or hypopnea or snoring during sleep, the airbags 69 and 70 are inflated, and thereby the lower jaw is elevated, and further the head is tilted backward, so that the airway can be reliably secured. Also, because the wearable body 64 is configured to be collar-like, freedom in the body posture during sleep is obtained, and no uncomfortable feeling will be given. Further, because the wearable body 64 has a simple structure including the collar-like body 68 and two airbags 69 and 70, the airway securing device 61 can be more compactly configured.

In the above-described example, the airway securing device 61 has included the airbag 69 for elevating the lower jaw, and the airbag 70 for tilting the head backward, however, the airway securing device 61 may be configured to include only the airbag 69 for elevating the lower jaw, omitting the airbag 70 for tilting the head backward. Alternatively, the airway securing device 61 may be configured to include only the airbag 70 for tilting the head backward while omitting the airbag 69 for elevating the lower jaw.

The airway securing devices in the above-described embodiments of the present invention aim to relieve snoring or normalize breathing of the patient suffering from sleep apnea syndrome. In the embodiments illustrated in FIG. 1, FIG. 3, FIG. 6, FIG. 7, FIG. 8A, FIG. 8B, FIG. 9A, and FIG. 9B, the airway securing devices can be applied to treatment and symptom improvement of sleep apnea syndrome. Also, in the embodiments illustrated in FIG. 6, FIG. 7, FIG. 8A, FIG. 8B, FIG. 9A, and FIG. 9B, the airway securing devices can be applied to relieving of snoring.

Further, the airway securing devices 1, 21, and 61 of the above-described first, second, and third embodiments can be applied to securing the airway during sleep due to anesthesia during a surgical operation.

The airway securing devices according to the first, second, and third embodiments employ a so-called airbag system, so that the devices are superior in weight (being light), compactness, flexibility, and so forth.

### [Fourth Embodiment]

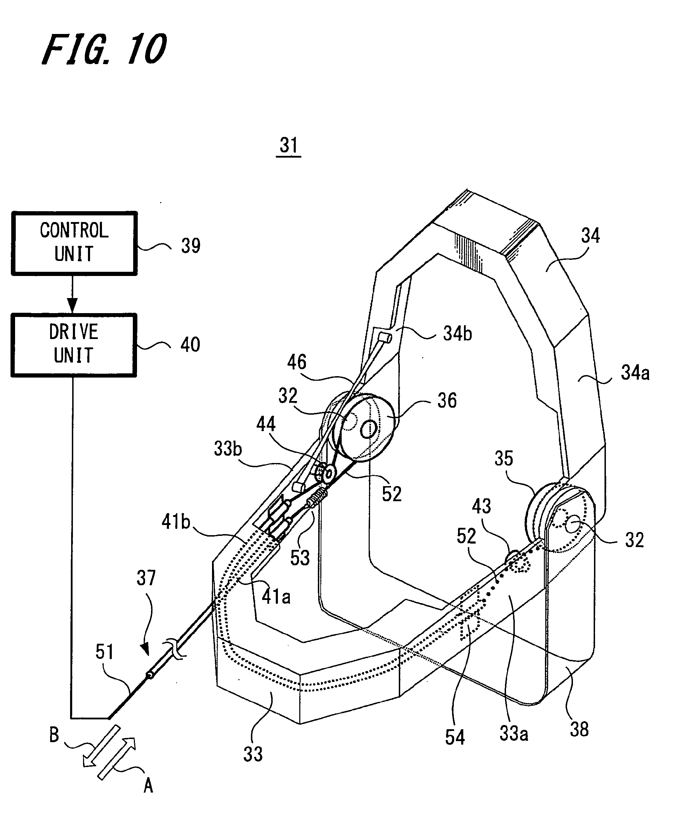

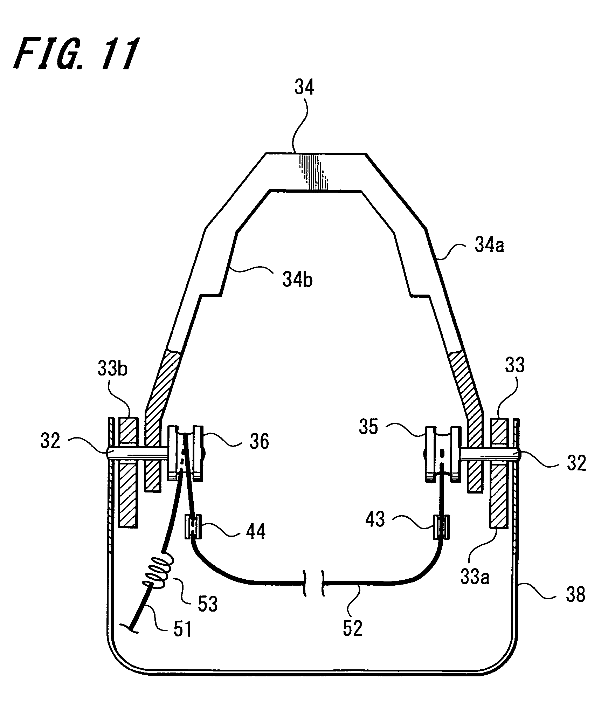

Referring to FIG. 10 and FIG. 11, the fourth embodiment of the airway securing device according to the present invention is described. An airway securing device 31 according to the present embodiment includes a lower frame 33 and an upper frame 34 supported by axes 32 at respective both ends thereof so as to open and close, a first pulley 35 and a second pulley 36 which are fixed to both ends of the upper frame 34, and a drive transmitting body 37 which is wound around the pulleys 35 and 36 to open and close the upper frame 34 relative to the lower frame 33. Also, a support frame 37 is attached to the lower frame 33 so that the upper frame 34 and the lower frame 33 will not move backward and forward relative to the neck of the patient. Further, the airway securing device 31 includes a control unit 39, and a drive unit 40 which drives the above-described wire 37.

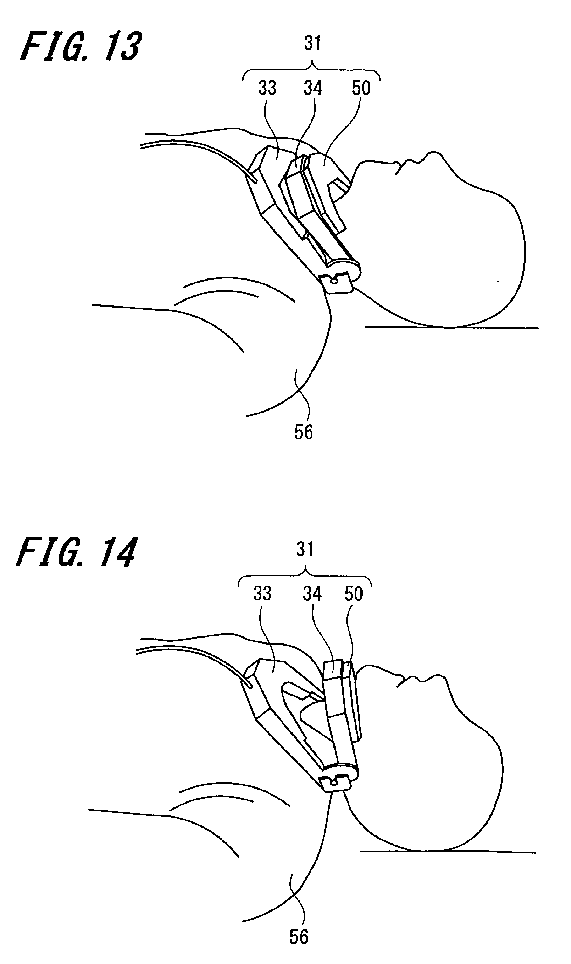

When the airway securing device 31 is worn around the neck of the patient, the lower frame 33 touches a front collar portion of pajamas, or in a naked body an upper portion of the chest corresponding to the front collar portion, and the upper frame 34 touches the lower jaw when the upper frame 34 is opened relative to the lower frame 33. The lower frame 33 and the upper frame 34 are each formed in a shape allowing a room relative to the neck when worn around the neck, in the present example, in a substantially U-shape. And, the axes 32 are fixedly provided at both ends of the substantially U-shaped upper frame 34, and both ends of the substantially U-shaped lower frame 33 are axially supported by the axes 32 in a rotatable manner. The both ends of the upper frame 34 are arranged inside the lower frame 33. A soft sheet 50 is attached to a surface of the upper frame 34, which touches the lower jaw, so that the lower jaw will not be injured (see FIG. 13 and FIG. 14).

The first pulley 35 is arranged to be fixed by press fitting, etc. to the axis 32 on one side provided at one end of the upper frame 34. The second pulley 36 is arranged to be fixed by press fitting, etc. to the axis 32 on the other side provided at the other end of the upper frame 34. Also, small pulleys 43 and 44 having smaller diameters than the first pulley 35 and the second pulley 36 are provided to internal surfaces of arm portions 33a and 33b of the U-shaped lower frame 33 close to the first pulley 35 and the second pulley 36, respectively.

The drive transmitting body 37 is configured by a wire 51 and a thread-like body (herein below, called a drive transmitting thread) 52. The wire 51 and the drive transmitting thread 52 are each configured to be partially inserted into a tube, and for example, Bowden cables 41a and 41b may be used. The wire 51 is inserted inside the lower frame 33 from the outside together with the tube, and is arranged along the internal surface of the arm portion 33b of the lower frame 33. The tip of the wire 51 coming out of the tube of the Bowden cable 41a is connected to the drive transmitting thread 52 through a buffering elastic body, in the present example, a buffer spring 53.

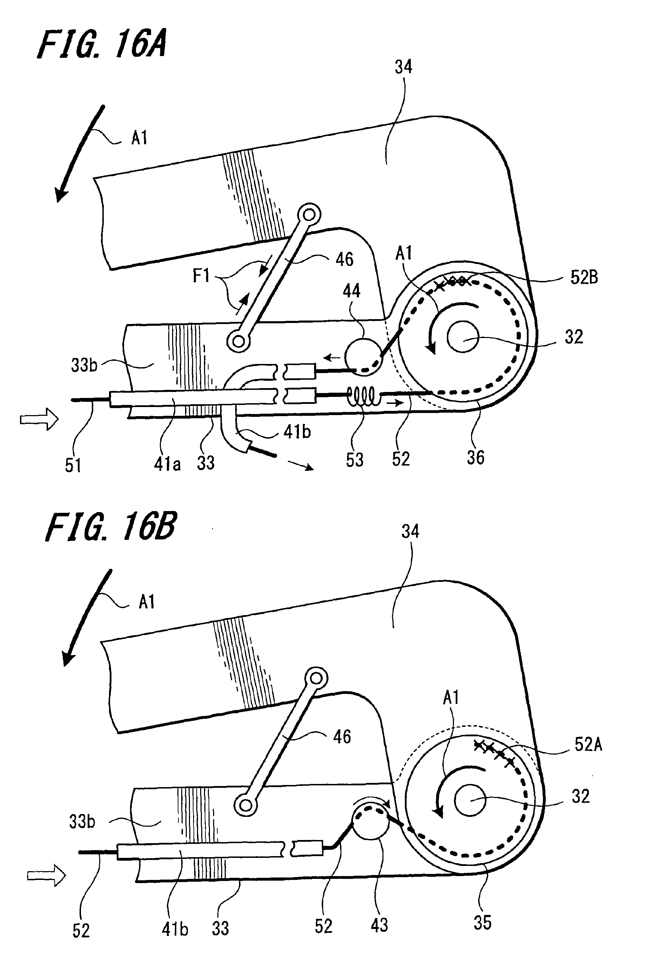

The drive transmitting thread 52 enters underneath the second pulley 36 and goes around to the upper side of the second pulley 36 to be wound around the second pulley 36, and after coming out from the upper side of the second pulley 36, the thread 52 is guided to the lower side of the small pulley 44, and then passes through the tube of the Bowden cable 41b, along the internal surface of the lower frame 33, to be arranged in the arm portion 33a on one side. A half-way portion 52B of the drive transmitting thread 52 is fixed to the second pulley 36 in the middle of being wound around the second pulley 36 (see FIG. 16A). The drive transmitting thread 52 coming out of the tube is wound around the first pulley 35 while being guided to the upper side of the small pulley 43 provided to the arm portion 33a on the one side. The drive transmitting thread 52 enters underneath the first pulley 35 and goes around to the upper side of the first pulley 35 to be wound around the first pulley 35, and a tip end (one end of the drive transmitting thread) 52A thereof is fixed to the first pulley 35 (see FIG. 16B).

Tube ends of the Bowden cable 41b arranged in the internal surface of the lower frame 33 are fixed to the internal surface of the lower frame 33 through attachment tools 54, respectively.

An elastic body, in the present example, a restoring force generating rubber material 46, which always acts on the upper frame 34 such that the upper frame 34 operates in the closing direction relative to the lower frame 33, is attached to the lower frame 33 and the upper frame 34, for example, to an internal surface of the arm portion 33b on the other side of the lower frame 33 and an internal surface of an arm portion 34b on the other side of the upper frame 34, so as to be spanned therebetween.

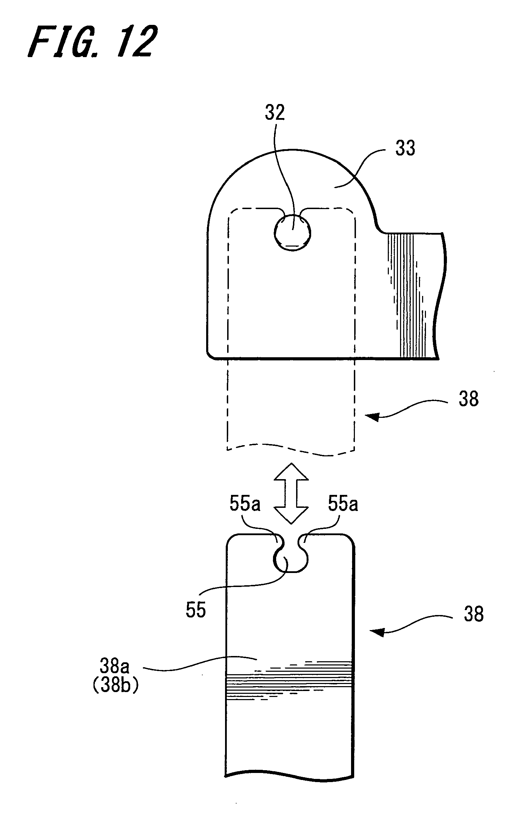

The support frame 38 is formed in a U-shape, and is detachably attached to the lower frame 33. For example, as illustrated in FIG. 12, the support frame 38 is arranged so as to sandwich both ends of the lower frame 33, and has cutouts 55 at tip ends of arm portions 38a and 38b. The cutout 55 has eaves so that the axes 32 can be rotated and resiliently held. The support frame 38 is attached to the lower frame 33 by fitting the cutouts 55 to the axes 32 from below. Also, the support frame 38 can be detached from the lower frame 33 by being pulled down.

Meanwhile, the drive unit 40 is constituted by an electric drive device such as an electric motor, etc. When an electric motor is used, an end of the wire 51 is connected to a drive transmitting portion connected to the electric motor, and for example, by controlling the revolving direction of the electric motor, the pushing and pulling operation of the wire 51 can be carried out.

The control unit 39 outputs necessary drive signals to the drive unit 40 based on input of information (signal) that airway securing is necessary or information (signal) that the condition is normal. For example, based on signals obtained at the previously-described sensor 4, whether the airway is secured is determined, and an instruction is sent from the control unit 39 to the drive unit 40.

For example, in treatment of snoring or sleep apnea syndrome, whether snoring or apnea and/or hypopnea is present during sleep is determined based on signals obtained at the sensor 4, and instructions are sent from the control unit 39 to the drive unit 40. Also, in the case of securing the airway during sleep due to anesthesia during a surgical operation, upon turning on the switch to start airway securing by the doctor or nurse, the control unit 39 sends an instruction to the drive unit 40, and the airway securing operation is carried out. The airway securing operation is continued, while confirming that airway closure is not present based on signals obtained at the sensor 4, until the stop switch is turned on. If by any chance an abnormality is found in breathing in the middle, it is communicated to the doctor by means of a warning sound, etc.

Next, the operation of the airway securing device 31 of the fourth embodiment is described. As illustrated in FIG. 13 and FIG. 14, in a condition that the patient lies, the airway securing device 31 is worn at the front collar portion so that open sides of the frames 33 and 34 oppose the neck. That is, the airway securing device 31 is worn with the lower frame 33 touching the front collar portion of pajamas and the upper frame 34 opposing the lower jaw. When the airway securing device 31 is worn on a naked body, the airway securing device 31 is worn on an upper part of the chest corresponding to the front collar portion of pajamas.

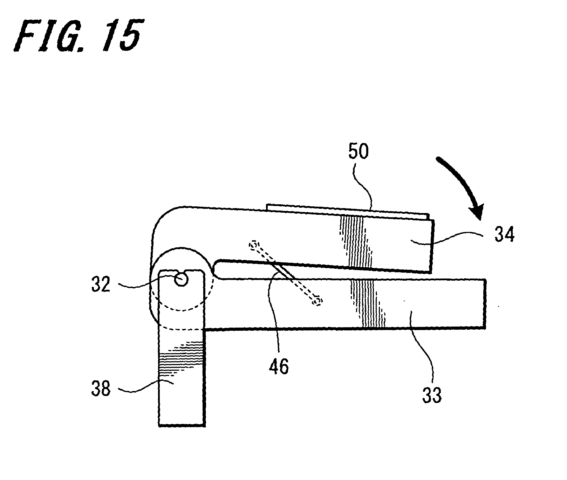

FIG. 13 illustrates a wearing condition at a normal time. At a normal time, the airway securing device 31 is, as illustrated in FIG. 13, in a condition that the upper frame 34 is closed relative to the lower frame 33. At a normal time, in FIG. 10, the wire 51 of the Bowden cable 41a is pushed in the arrow direction A by a drive force from the drive unit 40. Because of driving of the wire 51, the drive transmitting thread 52 wound around the second pulley 36 is loosened, and further the drive transmitting thread 52 wound around the first pulley 35 is loosened. At the same time, as illustrated in FIG. 16A and FIG. 16B, the restoration force generating rubber member 46 shrinks because of a restoring force F1, and thereby the upper frame 34 is rotated in the arrow direction A1 to be closed relative to the lower frame 33. At this time, with rotation of the upper frame 34 in the closing direction, the second pulley 36 and the first pulley 35, which are integral with the upper frame 34, are rotated in the same direction (arrow direction A1), so that the drive transmitting thread 52 turns into a strained condition. FIG. 15 illustrates a condition that the upper frame 34 has been closed relative to the lower frame 33.

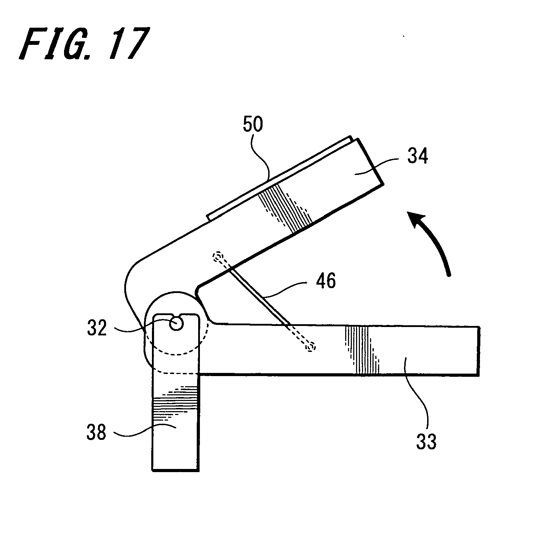

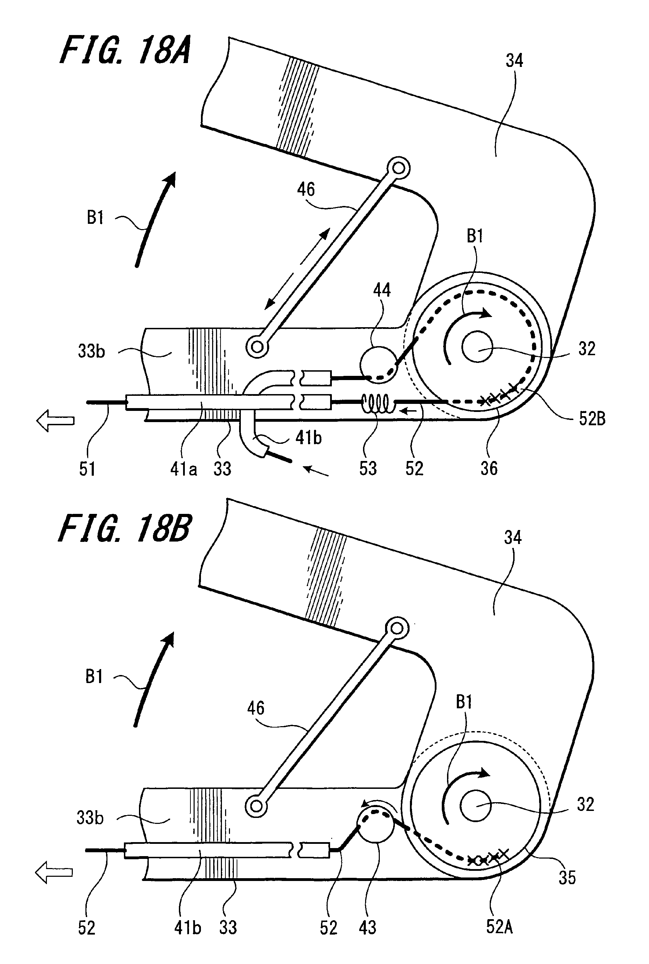

FIG. 14 illustrates a condition that the upper frame 34 is opened relative to the lower frame 33 and the lower jaw elevating operation has been carried out. At the time of the lower jaw elevating operation, in FIG. 10, the wire 51 is pulled in the arrow direction B by a drive force from the drive unit 40. Because of driving of the wire 51, as illustrated in FIG. 18A and FIG. 18B, the drive transmitting thread 52 wound around the second pulley 36 causes the second pulley 36 to rotate in the arrow direction B1. This rotating force is transmitted by the Bowden cable 41 in the lower frame 33 to the first pulley 35 through the small pulleys 44 and 43, and causes the first pulley 35 to rotate in the arrow direction B1. With rotation of the pulleys 35 and 36, the upper frame 34 is opened relative to the lower frame 33. At this time, the restoration force generating rubber member 46 is stretched while resisting the restoring force F1, and the upper frame 34 is put in an opened condition. The upper frame 34 opens while touching the lower jaw of the patient, so that the jaw tip is elevated, and the head is tilted backward, and thereby the airway space is expanded, and the airway can be secured. That is, because of the opening of the upper frame 34, the lower jaw elevating operation and the head backward tilting operation are carried out. FIG. 17 illustrates a condition that the upper frame 34 has been opened.

The buffer spring 53 aims realizing a comfortable lower jaw elevating operation and securing safety. By interposing the buffer spring 53, it is possible to make opening and closing of the upper frame 34 flexible. Incidentally, if the buffer spring 53 is not present between the wire 51 and the drive transmitting thread 52, the lower jaw is surely elevated as much as the wire 51 is driven. However, when a situation arises in which the lower jaw cannot be elevated any more for any reason, there is a possibility that a pain is caused by receiving an excessive force. In the present embodiment, by interposing the buffer spring 53, a cushion effect is exerted, so that the force gently acts, and the lower jaw is comfortably elevated. Even if by any chance a situation is caused in which the lower jaw cannot be elevated, the force equal to or greater than the restoring force which the buffer spring 53 can generate will not be applied, and it is safe.

According to the airway securing device 31 according to the fourth embodiment, ends of the upper frame 34, which correspond to the left and right joint portions, are driven at the same time, through the first and second pulleys 35 and 36, by the operation of one Bowden cable 41 from the outside. Accordingly, the airway securing device 31 can carry out the opening and closing operation of the upper frame 34 by exerting forces evenly on both sides thereof in a balanced manner to stabilize the upper frame 34, so that the stable lower jaw elevating operation and head backward tilting operation can be realized, and thereby the airway can be secured.

The airway securing device 31 of the fourth embodiment 31 can be used without requiring any specific body posture during sleep and without giving an uncomfortable feeling in wearing if slimming, such as omitting the support frame 38, etc., is tried, and if, for example, the lower frame 33 is fixed to the neckline of pajamas by a Velcro Tape (registered trademark).

The airway securing device 31 of the fourth embodiment, being an electrically-operated model which, upon detecting airway closure, secures the airway by being electrically driven, has advantages that the operation speed is fast and that it is easy to be produced. The airway securing device 31 requires a little bit larger drive force for realizing both of lower jaw elevating and head backward tilting operations. On the other hand, depending on patients, there is a case that tilting the head backward is not necessary and a sufficient effect is exerted on securing the airway only by elevating the lower jaw. In such a case, only a minor drive force is required, and as a result, an airway securing device which is very compact and cheap can be realized.

The airway securing device 31 of the fourth embodiment can be used as a treatment apparatus for snoring and sleep apnea syndrome. Also, the airway securing device 31 can be applied to securing the airway during sleep due to anesthesia during a surgical operation.

### Explanation of Reference Symbols

- 1, 21:: airway securing device for snoring or sleep apnea syndrome
- 2:: body
- 3:: wearable body
- 4:: sensor
- 5:: mechanism to close the mouth and elevate the jaw tip
- 6:: mechanism to tilt the head backward
- 7, 8, 27:: pouch-like body
- 11:: belt-type abdomen sensor unit
- 12:: belt-type chest sensor unit
- 14:: control unit
- 15:: drive unit
- 16:: pillow
- 28:: microphone
- 31:: airway securing device
- 32:: axis
- 33:: lower fame
- 34:: upper frame
- 35, 36:: pulley
- 37:: drive transmitting body
- 38:: support frame
- 39:: control unit
- 40:: drive unit
- 41:: Bowden cable
- 43, 44:: small pulley
- 46:: restoration force generating rubber
- 50:: soft sheet
- 51:: wire
- 52:: drive transmitting thread
- 53:: buffer spring
- 54:: attachment tool
- 55:: cutout
- 61:: airway securing device
- 62:: mechanism to close the mouth and elevate the jaw tip
- 63:: mechanism to tilt the head backward
- 64:: wearable body
- 67:: neck
- 68:: collar-like body
- 69:: airbag for elevating the lower jaw
- 70:: airbag for tilting the head backward
- 73:: pillow

## Claims

1. An airway securing device for securing an airway of a patient, comprising:
a wearable body configured to be worn on the patient;
and,
a mechanism provided to the wearable body and configured to close a mouth of the patient and elevate a jaw tip of the patient.

2. The airway securing device according to claim 1, further comprising a mechanism provided to the wearable body and configured to tilt a head of the patient backward.

3. The airway securing device according to claim 1 or claim 2, further comprising a mechanism provided to the wearable body and configured to detect an abnormality in the patient during sleep.

4. The airway securing device according to claim 3, wherein the mechanism to detect an abnormality in the patient during sleep includes either or both of a device detecting apnea and/or hypopnea and a device detecting occurrence of snoring.

5. The airway securing device according to claim 1, wherein the mechanism to close the mouth and elevate the jaw tip is formed by a pouch-like body which is inflated and deflated by air.

6. The airway securing device according to claim 2, wherein the mechanism to close the mouth and elevate the jaw tip and the mechanism to tilt the head backward are formed by pouch-like bodies which are inflated and deflated by air, respectively.

7. The airway securing device according to claim 3, wherein the wearable body is formed by clothing.

8. An airway securing device for securing an airway of a patient, comprising:
a wearable body having a collar-like body and configured to be worn on the patient; and
a pouch-like body provided at a front upper surface of the collar-like body of the wearable body and configured to be inflated and deflated by air to serve as a mechanism to close a mouth of the patient and elevate a jaw tip of the patient.

9. The airway securing device according to claim 8, further comprising a pouch-like body provided at a rear back surface of the collar-like body of the wearable body and configured to be inflated and deflated by air to serve as a mechanism to tilt a head of the patient backward.

10. The airway securing device according to claim 8 or claim 9, further comprising a mechanism provided in the collar-like body of the wearable body and configured to detect an abnormality in the patient during sleep.

11. An airway securing device for securing an airway of a patient, comprising:
a lower frame and an upper frame axially supported at respective both ends thereof so as to open and close, the upper frame touching a lower jaw of the patient when opened relative to the lower frame;
a first pulley and a second pulley fixed at both ends of the upper frame; and
a drive transmitting body fixed to the first pulley at one end thereof and wound around the first pulley and the second pulley, and configured to open and close the upper frame relative to the lower frame by a pushing and pulling operation thereof.

12. The airway securing device according to claim 11, further comprising:
an elastic body attached to the upper frame and the lower frame so as to be spanned therebetween and configured to always act on the upper frame such that the upper frame is moved in a closing direction relative to the lower frame; and
a buffering elastic body inserted in a part of the drive transmitting body on the second pulley side.

13. The airway securing device according to claim 12, further comprising a support frame attached to the lower frame and configured to prevent the upper frame and the lower frame from moving relative to a neck of the patient.

14. The airway securing device according to claim 11, 12 or 13, further comprising a drive device connected to the drive transmitting body and configured to push and pull the drive transmitting body.

15. The airway securing device according to claim 11, wherein each of the upper frame and the lower frame is formed in a substantially U-shape.
